# NEW EUROPEAN PATENT SPECIFICATION

(11) **EP 2 482 872 B2**
(45) Date of publication and mention of the opposition decision: **11.12.2019**
(45) Mention of the grant of the patent: 18.05.2016
(21) Application number: 10757784.3
(22) Date of filing: 29.09.2010
(51) Int. Cl.: A61M 5/20, A61M 5/24, A61M 5/315, A61M 5/31

(54) **METHOD FOR ASSEMBLING A DRUG DELIVERY DEVICE, ASSEMBLY FOR A DRUG DELIVERY DEVICE AND PISTON ROD FOR A DRUG DELIVERY DEVICE**
Verfahren zur Montage einer Arzneimittelabgabevorrichtung, Anordnung für eine Arzneimittelabgabevorrichtung und Pleuelstange für eine Arzneimittelabgabevorrichtung
Procédé pour l'assemblage d'un dispositif d'administration de médicaments, assemblage pour un dispositif d'administration de médicaments et tige de piston pour un dispositif d'administration de médicaments

(30) Priority: 30.09.2009 EP 09171757
(43) Date of publication of application: 08.08.2012
(73) Proprietor: Sanofi-Aventis Deutschland GmbH, 65929 Frankfurt am Main (DE)
(72) Inventor: MACDONALD, Catherine, Anne, Warwick Warwickshire CV34 6JE (GB); VEASEY, Robert, Leamington Spa Warwickshire CV32 7EP (GB); PLUMPTRE, David, Droitwich Spa Worcestershire WR9 7RQ (GB); JONES, Christopher, Tewkesbury Gloucestershire GL20 7AT (GB); KOUYOUMJIAN, Garen, Leamington Spa Warwickshire CV31 1HN (GB)
(74) Representative: Keil & Schaafhausen Patent- und Rechtsanwälte PartGmbB
(86) International application number: PCT/EP2010/064419
(87) International publication number: WO 2011/039226

(56) References cited:
- US-A- 5 085 640
- US-A1- 2004 254 526
- US-A1- 2006 052 748
- US-A1- 2006 173 409

## Description

The present invention relates to a method for assembling a drug delivery device, particularly for securing a cartridge to a body of a drug delivery device. The invention further relates to a drug delivery device, an assembly for a drug delivery device and a piston rod contained in an assembly for a drug delivery device.

Drug delivery devices are generally known to be used for the administration of medicinal products, for example insulin or heparin, but also for other medicinal products for self-administration by a patient. Often, drug delivery devices are pen-type injectors which dispense a pre-set dose of a fluid medicinal product.

Document US 2006/0052748 A1 discloses a single use syringe assembly comprising a plunger and a barrel with a fluid chamber as well as a proximal end and a distal end. The known syringe assembly includes a plunger locking mechanism, which when activated, locks the plunger in the barrel so that the syringe may not be reused.

Prior to the first use of the drug delivery device, the drug delivery device usually has to be primed. During a priming-step gaps may be closed which are contained in the drug delivery device between components, particularly between a piston rod and a cartridge bung, which are involved in the mechanism for dispense of the fluid medicinal product. These gaps may be a consequence of the tolerances associated with all the assembled parts which may occur through the manufacturing of the device and the requirement not to pre-load the bung axially in the assembled device. However, users who are not familiar with such pen-type injectors may fail to or incorrectly prime the drug delivery device before dispensing the first dose and may inject the prime fluid of an incorrect volume of the medicinal product delivered in the first dose.

It is an object of the present disclosure to provide an assembly for the use in a drug delivery device which is more user friendly and, particularly, helps to improve the accuracy of the first dispensed dose of the fluid medicinal product. This object may be achieved by the subject matter of the independent claims. Further features are the subject matter of dependent claims as well as the description.

According to a first aspect, a method for assembling a drug delivery device is provided. The method involves providing a cartridge retaining a bung and holding a drug as well as providing a body retaining a piston rod (step A). The body has a distal end and a proximal end which are spaced apart in the direction of an axis. The piston rod retained in the body is plastically deformable and at least a part of this plastically deformable piston rod is manufactured from a plastically deformable material. In a second step (step B), a force is applied on the piston rod in a state wherein at least a part of the piston rod or at least a part of the area being manufactured from a plastically deformable material allows plastic deformation. Upon applying the force on the piston rod, the piston rod is plastically deformed and an element of the piston rod abuts the bung. In a further step (step C), conditions are applied in order to bring the plastically deformed piston rod into an altered state wherein no further plastic deformation takes place. Before, during or after the step of plastic deformation, the cartridge is secured to the body (step D).

According to the present invention, a component is plastically deformable if this component is irreversibly deformed upon application of a force. Furthermore, the piston rod or the plastically deformable part or element of the piston rod undergoes plastic deformation without fracture or damage of the operational capability of the piston rod. After having plastically deformed the piston rod, conditions are applied which do not allow further plastic deformation, particularly, plastic deformation which would have occurred under the conditions of step B (i.e. occurred upon application of a force on the piston rod). Usually, these conditions do not involve fixing the plastically deformed piston rod to a further part of the drug delivery device or fixing the plastically deformed part and a further part of a multi-part piston rod to each other. Usually the conditions involve altering the physical state of matter or the chemical state of matter of at least a part of the material of the piston rod or both, the physical and the chemical state of matter. Additionally, the plastic deformation according to the present invention usually does not cause an increase of stresses or loads within the piston rod and usually also not an increase of stresses or loads within a one-piece or of a multi-part piston rod or a section thereof.

This method of assembling a drug delivery device according to the present invention solves the problem of risks associated with the prime set-up step by removing the need for the user to prime the pen injector before use. Therefore, the user does not have to prime the device and, therefore, will not accidentally inject prime fluid.

The method for assembling the drug delivery device according to the present invention allows adjusting the shape (particularly the axial extension) of the piston rod during assembly, preferably during final assembly after the medicament cartridge has been fitted so that an element of the piston rod abuts the bung retained in the cartridge. Due to the adjustment of the length of the piston rod, tolerances between components being responsible for disposing a dose of drug, particularly tolerances between the piston rod and the bung, are removed and the need for a "priming" operation to be undertaken by users prior to delivering the first dose of medicament is eliminated.

Usually, the piston rod provided in step A is intentionally too long to take up the maximum allowable gap between the components being involved in the mechanism to deliver a dose of drug, particularly the gap between the piston rod and the bung. By applying the force and thereby plastically deforming the piston rod, the distance extending between the distal end and the proximal end of the piston rod is adjusted in order to eliminate the gaps between aforesaid components.

Usually, the applied force results in a compression of the piston rod in longitudinal direction; however, also an expansion of the plastically deformable piston rod may be carried out at first followed by a compression as described before. If at first an expansion takes place, the length of the piston rod does not have to be intentionally too long to take up the maximum allowable gap between the aforesaid components of the assembly.

In an embodiment, the drive mechanism comprised in the body of the drug delivery device is brought into a position which resembles the situation during dispense of a dose of the drug or being identical with the situation during dispense of a dose of the drug prior to step B, particularly the situation at the end of the dispense step. This gives rise to correctly take up tolerances of the drive mechanism (or more general tolerances or gaps between the components being involved in the mechanism to deliver a dose) during step B. Further, a drive mechanism being in this situation would simulate forces that would be seen during dispense of a dose.

In an embodiment, the applied force of step B results from a movement of the piston rod in distal direction or a movement of a component being fixed to the piston rod in distal direction (i.e. a movement which also causes movement of the piston rod in distal direction), or a movement of the cartridge in proximal direction, or a movement of a component being fixed to the cartridge (i.e. a component which also causes movement of the cartridge) in proximal direction.

By carrying out the method according to this embodiment, step B is easily carried out when two main parts of the housing of the drug delivery device, the body and a cartridge holder retaining the cartridge, are assembled. However, the method according to this embodiment may also be carried out in a separate step, the assembly of body and cartridge holder taking place later on.

In a further embodiment, between steps A and B of the method of the present invention, a step E is carried out: in step E, the plastically deformable material is brought into a plastically deformable state of matter by heating. More generally speaking, the step of this embodiment involves the change of a state of matter where no plastic deformation of the piston rod is possible to a state of matter where plastic deformation is possible. However, embodiments starting from a state where no plastic deformation is possible usually involve raising the temperature in order to bring the piston rod into the state of matter where plastic deformation is possible.

Usually, the plastically deformable material is heated to a temperature above the glass transition temperature of the material, particularly of only heating the area to be plastically deformed above the glass transition temperature of the material of this area. Further, the temperature is usually not raised above the melting temperature of the respective material (i.e. the plastically deformable material).

The heating of the plastically deformable material may be effected by direct heating (e.g. a heated nest on the assembly line where the drug delivery device is assembled or by a concentrated light source which allows precisely determining the area which should undergo plastic deformation) or by indirect heating (e.g. by induction or by a chemical reaction which involves the release of heat).

If at standard conditions the piston rod or the area to be plastically deformed is already in a plastically deformable state of matter, no heating is necessary (i.e. step E may be omitted). This may be the case, if in step C the plastically deformable material is brought into a state of matter where no plastic deformation takes place for example by a chemical reaction.

In a further embodiment, the method according to the invention involves providing a piston rod which is at least partially manufactured from a polymer, particularly a thermoplastic polymer. Therefore, the plastically deformable material of step A may comprise a thermoplastic polymer or consist of a thermoplastic polymer. The material may, for example, comprise PVC or PMMA. By using a thermoplastic polymer, the piston rod provided in step A may easily be obtained by injection molding.

In a further embodiment, the altered state of matter of step C of the method of the present invention is obtained by cooling or by effecting a chemical reaction of the plastically deformable material.

Upon cooling, particularly cooling below the glass transition temperature of the plastically deformable material, the state of matter changes and the piston rod is not plastically deformable any longer. If a step E (during which the temperature of the plastically deformable material had been raised) is carried out, the cooling step involves changing the state back to the original state being present before the heating step E was carried out.

On the other hand, an altered state of matter may also be effected by a chemical reaction. Particularly, a chemical reaction may involve cross-linking of polymer chains of an oligomeric or polymeric material contained in the plastically deformable material or a polymerization of the plastically deformable material (e.g. a polymerization of monomers, oligomers or monomeric groups contained in a polymer). This chemical reaction can, for example, be started by radiation (for example UV-light) or by raising the temperature (causing, for example, an addition polymerization or a radical polymerization using e.g. a temperature-sensitive starter). Additionally, the chemical reaction may involve a reaction as known from two component adhesives, particularly a reaction of a binder and a curing agent. Preferably, the binder imparts dimensional stability (at least dimensional stability to a certain extent) of the piston rod or the plastically deformable material already in the state of matter before carrying out the chemical reaction. As far as cross-linking is concerned, particularly a change from a thermoplastic polymer to a duroplastic polymer may be effected by the chemical reaction.

In order to allow the production of a plastically deformable piston rod to be provided in step A which is already in the state where plastic deformation is possible, the material to be used for this piston rod usually needs to be dimensionally stable and should only be intentionally deformed during the deformation step B but not by other steps being involved in the assembly of the drug delivery device. Therefore, materials to be polymerized will usually contain oligomers, polymers or other components imparting an increased dimensional or mechanical stability (for example fillers).

In a further embodiment, the piston rod provided in step A of the present invention comprises an inductively heatable element or material.

Using a piston rod comprising an inductively heatable material (or compound) allows precisely predetermining the area which should undergo plastic deformation. Upon inductively heating a piston rod comprising an inductively heatable material, only the area comprising this material changes its state of matter into a state where plastic deformation may take place. Therefore, by adding inductively heatable material to the material of the piston rod, particularly to a piston rod made of thermoplastic material, a predetermined area of weakness may easily be defined.

The inductively heatable material may particularly be a ferro-magnetic material, for example in the form of iron particles. The inductively heatable material may be just mixed with the main material of the piston rod; it may also be chemically bonded to the main material of the piston rod, particularly the thermoplastic material, for example by using Fe₃O₄ particles with a chemically modified surface (giving rise to a more homogenous distribution of the inductively heatable material).

In a further embodiment, the piston rod comprises at least two elements, at least the first element comprising the plastically deformable material and at least a part of the second element being manufactured from an inductively heatable material. Usually, the first and the second element are abutting. Therefore, the second element may consist of an inductively heatable material; it also may comprise the inductively heatable material, for example the surface of the second element may completely or partially be covered by an inductively heatable material. However, any part of the second element may contain the inductively heatable material as long as the remaining part of the second element is manufactured from a material conducting heat. As mentioned before, the inductively heatable material is usually a ferro-magnetic material, and may be for example iron.

At least one of aforesaid first and second element in the assembled drug delivery device usually abuts the bung retained in the cartridge. However, also a further element abutting the bung may be present between the bung and the first and the second element.

The second element or also a further element of the piston rod may, for example, be a bearing being arranged between the main part of the piston rod and the bung, the bearing being particularly responsible for a transformation of any movement of the main part of the piston rod into a movement of the bung in distal direction only.

However, the second element may also be an element of any conceivable shape being comprised in the piston rod (for example with the shape of a ball); the only duty of such a second element being to transfer heat to the predetermined area of weakness comprised in the piston rod or in other words to the area comprising the plastically deformable material.

Upon inductively heating the second element, the state of matter of the plastically deformable material of the first element of the piston rod changes and thereby, the material is brought into a state of matter which allows carrying out step B.

According to a second aspect of the present invention, a further method for assembling a drug delivery device is provided.

In the method according to this aspect, a component is provided which defines the distance between the proximal face of the bung retained in the cartridge which holds a drug and the proximal end of the piston rod retained in the body (wherein the body has a distal end and a proximal end) in step A'. In steps B' and C', the cartridge and the distance defining component are engaged and the cartridge is arranged in its most distal position with respect to the distance defining the component. Subsequently, the distance between the proximal face of the bung and the proximal end of the distance defining component is determined. In step D', the gaps between the proximal face of the bung and the distal end of the piston rod is calculated from the distance determined in step C'. Subsequently, in step E', the length of the piston rod is adjusted to the length derived by the calculation obtained in step D'. Finally, the cartridge comprising the bung is secured to the body comprising the piston rod (step F').

Like the method according to the first aspect, the method according to the second aspect removes tolerances from the mechanism and eliminates the need for a "priming" operation to be undertaken by users prior to delivering the first dose of medicament.

The adjusting of the length of the piston rod may involve any adjusting method or step being described for the method according to the first aspect, particularly by adjusting the length of the piston rod by plastic deformation. However, also other adjusting methods like cutting (e.g. laser cutting) may be applied.

According to an embodiment, the distance defining component of this aspect may be a cartridge holder or the piston rod.

If there is also tolerance between the proximal end of the cartridge holder being used as distance defining component and the proximal end of the piston rod, also the distance between the piston rod in its most proximal position with respect to the cartridge holder is usually taken into account in the calculation step D'.

According to a third aspect, a piston rod contained in an assembly of a drug delivery device is provided. The piston rod has a distal end and a proximal end which are spaced apart in the direction of an axis and comprises a predetermined area of weakness. This piston rod is deformable upon application of a force, particularly in the direction of the axis with respect to the piston rod.

The piston rod according to the third aspect allows easy adjustment of the length of the piston rod, particularly if the material comprised in the predetermined area of weakness is in a plastically deformable state of matter.

In particular, the piston rod may be constructed as described before. Particularly, the predetermined area of weakness preferably is an area which comprises or consists of plastically deformable material. Further, the predetermined area of weakness is usually not designed to allow mechanical engagement of two parts of the piston rod during step B, particularly not for connecting the two parts in a form-fitting way.

In an embodiment, the predetermined area of weakness (or in general the piston rod) comprises one or more openings or being more general one or more recesses being present in the piston rod. Therefore, the predetermined area of weakness usually comprises areas which are designed for changing their shape upon application of the force in axial direction. Particularly, the recesses or openings may have a shape which allows an easier deformation by a force applied in the direction of the axis than a force applied, for example, perpendicularly to this axis. For example, the surface of the predetermined area of weakness may comprise one or more recesses in the shape of a fold, for example a circumferential fold with respect to aforesaid axis. Openings may have a shape where the distance of the opening in the direction of the axis is longer than the distance in the direction perpendicular to the axis. Upon application of the force, the size of the opening or the recess may decrease, for example due to the deformation (i.e. the opening takes up deformed material).

Usually, the method involving the adjustment of the length of the piston rod is independent of the main mechanism used to set and dispense drug doses with the drug delivery device. Therefore, the form of the piston rod used for this invention is arbitrary as long as the piston rod comprises a plastically deformable area. Further, the area of the piston rod to be plastically deformed usually does not overlap with areas of the piston rod being responsible for the main mechanism for setting and dispensing drug doses (and does for example not overlap with parts of the piston rod interacting with components of the drive mechanism). Therefore, the plastically deformed area usually does not comprise parts of the piston rod being necessary for the mechanism of setting and dispensing drug doses.

According to a fourth aspect, an assembly for a drug delivery device is provided. The assembly comprises a body retaining a piston rod, particularly the piston rod as described before, and a cartridge retaining a bung. The cartridge is secured to the body and an element (i.e. the part at the distal end) of the piston rod abuts the proximal face of the bung. The piston rod comprises a plastically deformed element comprising a plastically deformed area. At least a part of said plastically deformed element is obtained from a plastically deformable material.

The element of the piston rod comprising the plastically deformed area may be the element of the piston rod abutting the bung.

Usually, the plastically deformed area is characterized by features derived from a compression of an predetermined area of weakness. Preferably, the assembly according to this aspect is obtained by one of the methods described before.

According to a fifth aspect, a drug delivery device comprising an assembly as described before is provided.

The drug delivery device may be an injection device. The drug delivery device may be a pen-type device, e.g. a pen-type injector which may be an injector for single-use or multiple-use. The cartridge may hold a plurality of doses of a drug. Preferably, the drug comprises a liquid medication, such as a long-acting or short-acting insulin, GLP-1, heparin or growth hormones. The drug delivery device may be designed such that it may accommodate cartridges of different sizes. Additionally or alternatively, the drug delivery device may be designed such that it may accommodate cartridges of different shapes.

The cartridge/cartridge holder may be permanently secured to the body by connection means. For example, the connection means may be joined by welding. Additionally or alternatively, the connection may comprise use of a separate connecting material such as an adhesive. The cartridge holder may be reversibly or irreversibly secured to the body, alternatively, the cartridge may be directly secured to the body and the use of a cartridge holder may be redundant.

The term "drug", as used herein, preferably means a pharmaceutical formulation containing at least one pharmaceutically active compound,
wherein in one embodiment the pharmaceutically active compound has a molecular weight up to 1500 Da and/or is a peptide, a proteine, a polysaccharide, a vaccine, a DNA, a RNA, an enzyme, an antibody, a hormone or an oligonucleotide, or a mixture of the above-mentioned pharmaceutically active compound,
wherein in a further embodiment the pharmaceutically active compound is useful for the treatment and/or prophylaxis of diabetes mellitus or complications associated with diabetes mellitus such as diabetic retinopathy, thromboembolism disorders such as deep vein or pulmonary thromboembolism, acute coronary syndrome (ACS), angina, myocardial infarction, cancer, macular degeneration, inflammation, hay fever, atherosclerosis and/or rheumatoid arthritis,
wherein in a further embodiment the pharmaceutically active compound comprises at least one peptide for the treatment and/or prophylaxis of diabetes mellitus or complications associated with diabetes mellitus such as diabetic retinopathy,
wherein in a further embodiment the pharmaceutically active compound comprises at least one human insulin or a human insulin analogue or derivative, glucagon-like peptide (GLP-1) or an analogue or derivative thereof, or exedin-3 or exedin-4 or an analogue or derivative of exedin-3 or exedin-4.

Insulin analogues are for example Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin. Insulin derivates are for example B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl-ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω-carboxyheptadecanoyl) human insulin.

Exendin-4 for example means Exendin-4(1-39), a peptide of the sequence H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-NH2.

Exendin-4 derivatives are for example selected from the following list of compounds:
H-(Lys)4-des Pro36, des Pro37 Exendin-4(1-39)-NH2,
H-(Lys)5-des Pro36, des Pro37 Exendin-4(1-39)-NH2,
des Pro36 [Asp28] Exendin-4(1-39),
des Pro36 [IsoAsp28] Exendin-4(1-39),
des Pro36 [Met(0)14, Asp28] Exendin-4(1-39),
des Pro36 [Met(0)14, IsoAsp28] Exendin-4(1-39),
des Pro36 [Trp(O2)25, Asp28] Exendin-4(1-39),
des Pro36 [Trp(02)25, IsoAsp28] Exendin-4(1-39),
des Pro36 [Met(0)14 Trp(02)25, Asp28] Exendin-4(1-39),
des Pro36 [Met(0)14 Trp(02)25, IsoAsp28] Exendin-4(1-39); or
des Pro36 [Asp28] Exendin-4(1-39),
des Pro36 [IsoAsp28] Exendin-4(1-39),
des Pro36 [Met(0)14, Asp28] Exendin-4(1-39),
des Pro36 [Met(0)14, IsoAsp28] Exendin-4(1-39),
des Pro36 [Trp(O2)25, Asp28] Exendin-4(1-39),
des Pro36 [Trp(02)25, IsoAsp28] Exendin-4(1-39),
des Pro36 [Met(0)14 Trp(02)25, Asp28] Exendin-4(1-39),
des Pro36 [Met(0)14 Trp(02)25, IsoAsp28] Exendin-4(1-39),
wherein the group -Lys6-NH2 may be bound to the C-terminus of the Exendin-4 derivative;
or an Exendin-4 derivative of the sequence
H-(Lys)6-des Pro36 [Asp28] Exendin-4(1-39)-Lys6-NH2,
des Asp28 Pro36, Pro37, Pro38Exendin-4(1-39)-NH2,
H-(Lys)6-des Pro36, Pro38 [Asp28] Exendin-4(1-39)-NH2,
H-Asn-(Glu)5des Pro36, Pro37, Pro38 [Asp28] Exendin-4(1-39)-NH2,
des Pro36, Pro37, Pro38 [Asp28] Exendin-4(1-39)-(Lys)6-NH2,
H-(Lys)6-des Pro36, Pro37, Pro38 [Asp28] Exendin-4(1-39)-(Lys)6-NH2,
H-Asn-(Glu)5-des Pro36, Pro37, Pro38 [Asp28] Exendin-4(1-39)-(Lys)6-NH2,
H-(Lys)6-des Pro36 [Trp(02)25, Asp28] Exendin-4(1-39)-Lys6-NH2,
H-des Asp28 Pro36, Pro37, Pro38 [Trp(02)25] Exendin-4(1-39)-NH2,
H-(Lys)6-des Pro36, Pro37, Pro38 [Trp(02)25, Asp28] Exendin-4(1-39)-NH2,
H-Asn-(Glu)5-des Pro36, Pro37, Pro38 [Trp(02)25, Asp28] Exendin-4(1-39)-NH2,
des Pro36, Pro37, Pro38 [Trp(02)25, Asp28] Exendin-4(1-39)-(Lys)6-NH2,
H-(Lys)6-des Pro36, Pro37, Pro38 [Trp(02)25, Asp28] Exendin-4(1-39)-(Lys)6-NH2,
H-Asn-(Glu)5-des Pro36, Pro37, Pro38 [Trp(02)25, Asp28] Exendin-4(1-39)-(Lys)6-NH2,
H-(Lys)6-des Pro36 [Met(0)14, Asp28] Exendin-4(1-39)-Lys6-NH2,
des Met(0)14 Asp28 Pro36, Pro37, Pro38 Exendin-4(1-39)-NH2,
H-(Lys)6-desPro36, Pro37, Pro38 [Met(0)14, Asp28] Exendin-4(1-39)-NH2,
H-Asn-(Glu)5-des Pro36, Pro37, Pro38 [Met(0)14, Asp28] Exendin-4(1-39)-NH2,
des Pro36, Pro37, Pro38 [Met(0)14, Asp28] Exendin-4(1-39)-(Lys)6-NH2,
H-(Lys)6-des Pro36, Pro37, Pro38 [Met(0)14, Asp28] Exendin-4(1-39)-(Lys)6-NH2,
H-Asn-(Glu)5 des Pro36, Pro37, Pro38 [Met(0)14, Asp28] Exendin-4(1-39)-(Lys)6-NH2,
H-Lys6-des Pro36 [Met(0)14, Trp(02)25, Asp28] Exendin-4(1-39)-Lys6-NH2,
H-desAsp28 Pro36, Pro37, Pro38 [Met(0)14, Trp(02)25] Exendin-4(1-39)-NH2,
H-(Lys)6-des Pro36, Pro37, Pro38 [Met(0)14, Asp28] Exendin-4(1-39)-NH2,
H-Asn-(Glu)5-des Pro36, Pro37, Pro38 [Met(0)14, Trp(02)25, Asp28] Exendin-4(1-39)-NH2,
des Pro36, Pro37, Pro38 [Met(0)14, Trp(02)25, Asp28] Exendin-4(1-39)-(Lys)6-NH2,
H-(Lys)6-des Pro36, Pro37, Pro38 [Met(0)14, Trp(02)25, Asp28] Exendin-4(S1-39)-(Lys)6-NH2,
H-Asn-(Glu)5-des Pro36, Pro37, Pro38 [Met(0)14, Trp(02)25, Asp28] Exendin-4(1-39)-(Lys)6-NH2;
or a pharmaceutically acceptable salt or solvate of any one of the afore-mentioned Exedin-4 derivative.

Hormones are for example hypophysis hormones or hypothalamus hormones or regulatory active peptides and their antagonists as listed in Rote Liste, ed. 2008, Chapter 50, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

A polysaccharide is for example a glucosaminoglycane, a hyaluronic acid, a heparin, a low molecular weight heparin or an ultra low molecular weight heparin or a derivative thereof, or a sulphated, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium.

Pharmaceutically acceptable salts are for example acid addition salts and basic salts. Acid addition salts are e.g. HCl or HBr salts. Basic salts are e.g. salts having a cation selected from alkali or alkaline, e.g. Na+, or K+, or Ca2+, or an ammonium ion N+(R1 )(R2)(R3)(R4), wherein R1 to R4 independently of each other mean: hydrogen, an optionally substituted C1-C6-alkyl group, an optionally substituted C2-C6-alkenyl group, an optionally substituted C6-C10-aryl group, or an optionally substituted C6-C10-heteroaryl group. Further examples of pharmaceutically acceptable salts are described in "Remington's Pharmaceutical Sciences" 17. ed. Alfonso R. Gennaro (Ed.), Mark Publishing Company, Easton, Pa., U.S.A., 1985 and in Encyclopedia of Pharmaceutical Technology.

Pharmaceutically acceptable solvates are for example hydrates.

In an embodiment the drug delivery device is a fixed dose device. This means that the device always dispenses a pre-given, non-user-variable, e.g. constant or varying dose of drug. Therefore, the drug delivery device may, for example, be used for drugs which should always be administrated by the user in the same dose. Especially if the drug should always be dispensed in a fixed dose, it is expedient that the first dose has exactly the same volume as the following doses. In one embodiment the device is a pen-type injector.

The drug delivery device may be used with a pen injector for the delivery of doses from a cartridge into the body by means of a needle. The injector-pen may be a disposable pen, for example a disposable fixed-dose injector. However, the present invention is not limited to disposable fixed-dose injectors; also, variable dose pens and reusable devices are possible.

Of course, features relating to different aspects described above may be combined with each other. Further features, advantages and expediencies become apparent from the following description of the exemplary embodiments in conjunction with the accompanying drawings.
- FIG. 1: schematically shows a side view of an embodiment of a drug delivery device.
- FIG. 2: shows a schematic view of the distal end of a first embodiment of a piston rod.
- FIGS. 3A and 3B: show schematic views of a second embodiment of a piston rod before (Fig. 3A) and after (Fig. 3B) plastic deformation.
- FIGS. 4A and 4B: show schematic views of the distal end of a third embodiment of a piston rod before (Fig. 4A) and after (Fig. 4B) plastic deformation; in Fig. 4B the piston rod abuts a bung.
- FIGS. 5A and 5B: show schematic views of the distal end of a fourth embodiment of a piston rod before (Fig. 5A) and after (Fig. 5B) plastic deformation; in Fig. 5B the piston rod abuts a bung.
- FIG. 6: shows a view of the distal end of a piston rod according to a fifth embodiment, the piston rod comprising a bearing.
- FIG. 7: shows a view of the distal end of a piston rod according to a sixth embodiment comprising a bearing and an inductively heatable element.
- FIGS. 8A to 8C: show schematic views of the distal end of a seventh embodiment of a piston rod comprising a bearing before (Fig. 8A and Fig. 8B) and after (Fig. 8C) plastic deformation. Fig. 8B additionally shows the area of the piston rod brought into a plastically deformable state of matter by heating.

Elements of the same kind and identically acting elements may be provided with the same reference numerals in the figures.

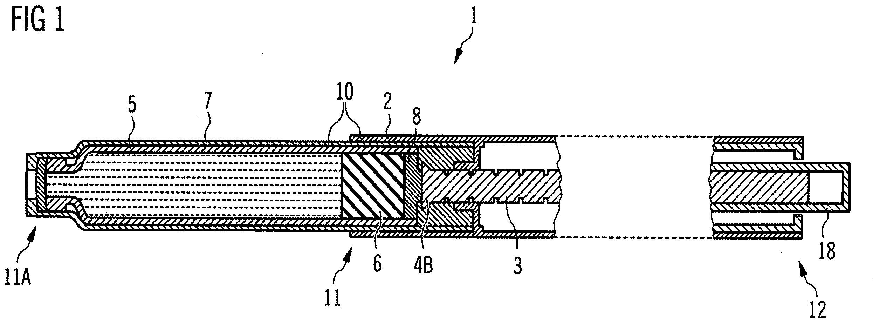

In FIG. 1, a drug delivery device 1 is shown. The drug delivery device comprises a body 2 and a cartridge holder 7, the body 2 and the cartridge holder 7 being elements of the housing 10. The housing 10 has a distal end 11 A and a proximal end 12; the body 2 has a distal end 11 and the same proximal end 12 as the housing 10. A cartridge 5 is located in the cartridge holder 7; the cartridge holder 7 may stabilize the cartridge 5 mechanically. The cartridge 5 contains a drug, preferably a plurality of doses of drug. The drug preferably comprises a liquid medication, for example insulin, e.g. short-acting or long-acting insulin, GLP-1, heparin or growth hormones.

The cartridge 5 may comprise an outlet (not explicitly shown) which may be covered by a membrane. The drug can be dispensed from the cartridge 5 through the outlet when the membrane is pierced. Further, the drug delivery device 1 may comprise means for securing a needle assembly (not explicitly shown) to the cartridge holder 7. The needle assembly may pierce the membrane when the drug delivery device 1 is operated. Operating the drug delivery device 1 (i.e. setting and dispensing a dose) involves movement of the dosing element 18.

The drug delivery device 1 comprises a piston rod 3 with a plastically deformed area 4B at its distal end. At the distal face of the piston rod, a bearing 8 is arranged, the bearing abutting the bung 6 retained in the cartridge 5. Particularly the bearing 8 is not contained in all embodiments according to the present invention.

The piston rod 3 provided for the method of assembling the drug delivery device 1 is initially usually located almost entirely within the body 2 and usually lies on the main axis of the drug delivery device 1. During assembly, the cartridge 5 is placed into the cartridge holder 7. The distal end of the piston rod 3 (more generally: the predetermined area of weakness 4A) is brought into (or is already in) a plastically deformable state of matter. The body 2 retaining the piston rod 3 is connected to the cartridge holder 7 comprising the cartridge 5. The drive mechanism retained in the body 2 of the drug delivery device 1 is loaded in order to simulate forces that would be seen during dose dispense in order to correctly take up tolerances of the mechanism during assembly. During this stage of final assembly, the piston rod 3 comes into contact with the bung 6 and the piston rod 3 (particularly the area of weakness 4A) controllably plastically deforms and the length of the piston rod 3 is adjusted. In this instance, the piston rod 3 is assembled such that it is under load as it contacts the bung 6. Finally, upon cooling (or after having carried out a chemical reaction), the piston rod 3 becomes rigid and now contains the plastically deformed area 4B and remains in contact with the bung 6. Any gap having been present between bung 6 and piston rod 3 is now removed.

The piston rod 3 may be of unitary or multi-part construction. Thus, the piston rod 3 may contain several elements or may be just a one-piece element. In a piston rod 3 containing two or more elements, just one element, two elements or even more elements may be necessary for the step of plastical deformation (thereby adjusting the length of the piston rod). In case of a multi-part construction usually the plastically deformed part and a further part or further parts of the piston rod are not designed to be permanently fixed to each other after step B has been carried out. Particularly, a bearing 8 may be one of the elements of the piston rod 3, the bearing facilitating interaction of the piston rod 3 and the bung 6.

The piston rod 3 is movable with respect to the cartridge 5. Movement of the piston rod 3 in distal direction with respect to the cartridge causes the drug to be dispensed from the cartridge through the outlet.

The housing 10 may be designed to enable a safe and comfortable handling of the drug delivery device 1. The housing 10 may be configured to house, fix, protect and guide inner components of the drug delivery device, e.g. piston rod 3 and dosing element 18. Preferably, the housing 10 limits or prevents exposure of the inner components to contaminants such a liquid, dirt or dust. The housing 10 may comprise a tubular or a cylindrical shape; alternatively, the housing 10 may comprise a non-tubular shape.

The drug delivery device 1 may be a pen-type device and may be disposable or reusable. The device may be configured to dispense fixed doses of the drug or variable, preferably user-settable doses of the drug. Particularly for a fixed dose device, it may be crucial that there is no gap between the piston rod and the bung.

The drug delivery device 1, particularly the body 2 may comprise a drive mechanism (not explicitly shown in the figures). The drive mechanism may be retained within the body 2. The specific mechanism for moving the piston rod in distal direction is omitted in FIG. 1 for the purpose of clarity as the mechanisms being relevant for the method for assembling the drug delivery device according to the present invention and the mechanisms being relevant for the method for setting and dispensing doses of the drug are usually independent from each other. During the set-and-dispense mode, the direction of movement of the piston rod may be a movement in distal direction only (in this embodiment, the bearing 8 is usually not present) or may also comprise the movement of the piston rod around its axis (the axis extending from the distal end to the proximal end).

When delivering a dose of the drug, due to an operation of the dosing element 18, a movement of the piston rod 3 in distal direction is caused. The user may displace the dose member 18 in the proximal direction with respect to the housing 10 for setting a dose of the drug. Afterwards, the user may displace the dosing element 18 in the distal direction with respect to the housing 10 for delivering the set dose of the drug.

The cartridge holder 7 and the body 2 may be adapted to releasably engage with each other or may be irreversibly fixed to each other (e.g. by means of an adhesive or mechanical clip). The cartridge holder 7 may be connectable to the body 2 of the drug delivery device 1, for example by means of a releasable connection.

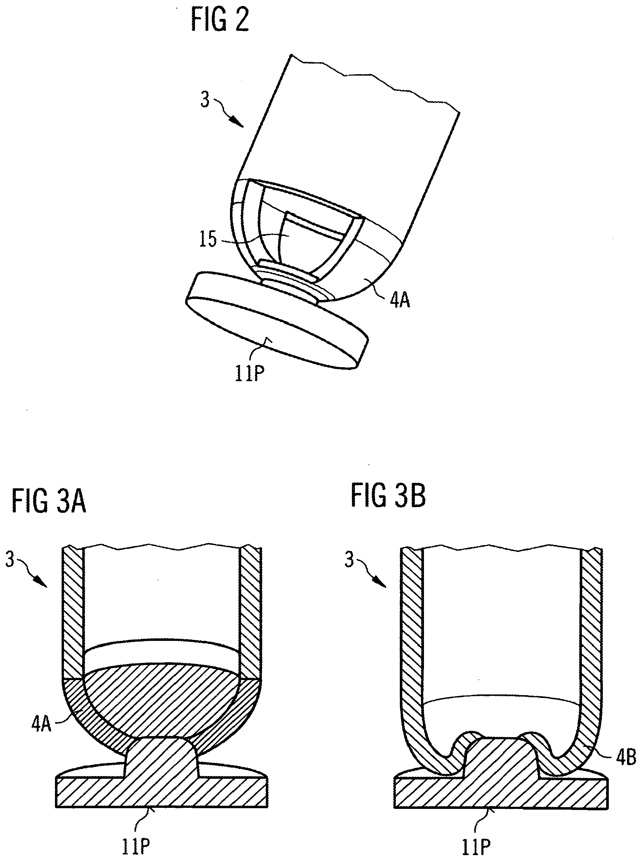

FIG. 2 shows the distal end of a first embodiment of a piston rod 3. The distal end comprises the distal face 11 P as well as a predetermined area of weakness 4A having a hemispherical geometry comprising two bars separated by two openings 15. The distal end of the piston rod 3 is designed so that upon applying a force in axial direction, the disk-type part of the piston rod comprising the distal face 11 P is pushed towards the main part of the piston rod and a plastic deformation of the predetermined area of weakness 4A having a hemispherical geometry takes place.

FIGS. 3A and 3B show the distal end of a second embodiment of a piston rod 3 being similar to the embodiment of FIG. 2. The piston rod 3 comprises a distal face 11 P and a predetermined area of weakness 4A shown in FIG. 3A. The piston rod 3 (or more precisely, the predetermined area of weakness 4A) is brought into a state of matter which allows plastic deformation (e.g. by heating, shown in FIG. 3A with a hatching rising rightwards). Upon applying a force in direction of the axis extending from the distal end to the proximal end of the piston rod 3 a deformation of the predetermined area of weakness 4A takes place. Upon this movement, again, the disk-shaped part of the piston rod comprising the proximal face 11 P is pushed towards the main part of the piston rod 3 upon which the predetermined area of weakness 4A is converted into the plastically deformed area 4B. This movement may involve a deformation of the bent parts of the predetermined area of weakness 4A so that the most distal part of the plastically deformed area 4B touches the disk-shaped component of the piston rod 3. Thereby, the step involving the plastic deformation of the piston rod 3 may give rise to an improved mechanical stability of the distal end of the piston rod 3.

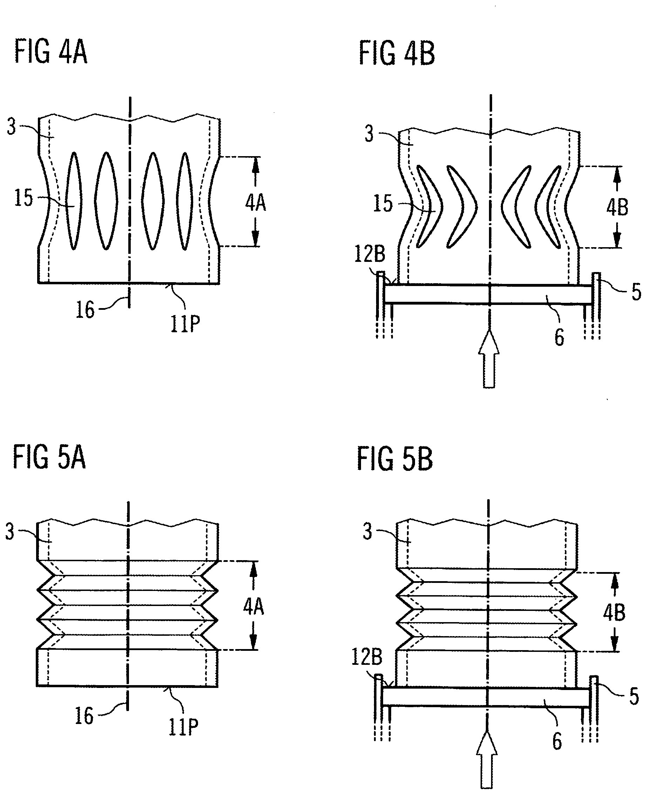

FIGS. 4A and 4B show the distal end of a third embodiment of a piston rod 3. The geometry of the piston rod is designed to encourage controlled, axial collapse of the distal end of the piston rod under load, particularly when the predetermined area of weakness 4A is heated. The geometry of the piston rod 3 according to this embodiment comprises a plurality of openings 15 having an extension in the direction of the axis 16 (being defined by the line spanned between the proximal end of the piston rod 3 and the distal face 11 P, particularly the center of the distal face 11 P of the piston rod) being much longer than the extension in the direction perpendicular to the axis 16 (resembling a lantern-like shape). FIG. 4B shows the situation after applying a force in the direction of the axis 16 (shown by an arrow) on the piston rod 3 being in a state of matter that allows plastic deformation. The extension of the openings 15 in the direction perpendicular to the axis 16 is shortened much more than the extension in the direction of the axis 16. The piston rod 3 abuts on the bung 6 retained in the cartridge 5, more precisely, the distal face 11 P of the piston rod 3 abuts on the proximal face 12B of the bung 6. The deformation of the predetermined area of weakness 4A gives rise to a plastically deformed area 4B having an extension in the direction of the axis 16 shortened with respect to the extension in the direction of the axis 16 of the predetermined area of weakness 4A.

FIGS. 5A and 5B show the distal end of a fourth embodiment of a piston rod 3 being designed to encourage controlled axial collapse of the piston rod under load. The piston rod according to this embodiment comprises a plurality of elements being more easily compressible by applying of a force in the direction of the axis 16 than a piston rod 3 containing no recesses. The predetermined area of weakness 4A in FIG. 5A has a bellowed shape. Upon application of a force in the direction of the axis (shown by an arrow in FIG. 5B), the proximal face 12B of the bung 6 abuts on the distal face 11 P of the piston rod 3. The piston rod 3 is, for example in a heated and plastically deformable state of matter and the area with bellowed shape is pushed so that the extension of the plastically deformed area 4B in the direction of the axis 16 is shortened compared to the extension of the plastically deformable area 4A of FIG. 5A.

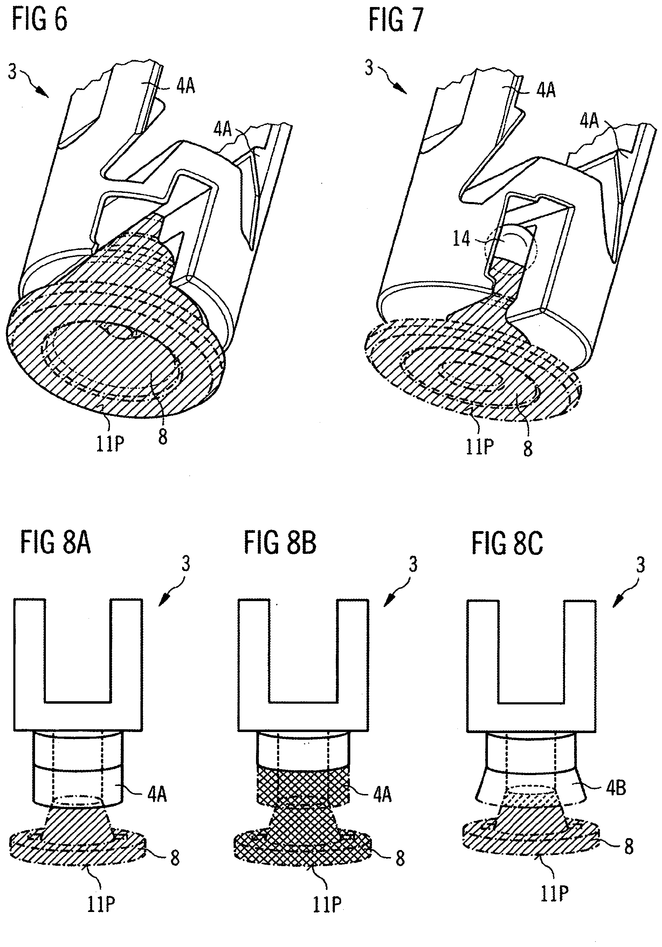

FIG. 6 shows the distal end of a fifth embodiment of a piston rod 3, the piston rod comprising at least two elements. The element arranged at the most distal part of the piston rod 3 comprising the distal face 11 P of the piston rod is constructed to be a bearing 8. The piston rod further comprises a predetermined area of weakness 4A.

FIG. 7 shows the distal end of a sixth embodiment of a piston rod 3, being similar to the embodiment shown in FIG. 6. Again, the distal face 11 P of the piston rod 3 is a part of the bearing 8. Between the distal end of the main part of the piston rod 3 (comprising the predetermined area of weakness 4A), an inductively heatable element 14 with a ball shape is located. The inductively heatable element 14 may, for example, be made of a metal and may comprise an inductively heatable material on its surface. However, also an inductively heatable core and a surface made from a heat transporting material are possible.

FIGS. 8A to 8C show the distal end of a seventh embodiment of a piston rod 3. The state of matter before plastic deformation takes place is depicted in FIG. 8A, the distal end of the piston rod 3 comprises a bearing 8, the bearing 8 comprising the distal face 11 P of the piston rod 3. The most distal part of the main element of the piston rod 3 comprises the predetermined area of weakness 4A.

In FIG. 8B, the step of selectively heating an area of the piston rod 3 is shown. The heated part of the piston rod 3 is designed with a checked pattern. For example, the bearing 8 may be made of a metal or even of an inductively heatable material; therefore, the predetermined area of weakness 4A may be heated by inductively heating the bearing 8; otherwise, heating of the predetermined area of weakness 4A, for example by means of a concentrated light source, may also involve heating a bearing 8 being made of a heat transporting material. If, however, the proximal face of the bung (not shown) is sensitive against heat, the lower part of the bearing 8 comprising the distal face 11 P of the piston rod 3 may be made of a material with a low heat conduction.

FIG. 8C shows the distal end of the piston rod 3 after plastic deformation. The distal face 11 P of the piston rod 3 abuts on the proximal face of the bung (not shown) and the main element of the piston rod 3 is pushed towards the bearing 8 having the effect that the predetermined area of weakness 4A is plastically deformed and that the diameter of the predetermined area of weakness 4A (in the direction perpendicular to the axis) is widened; the plastically deformed area 4B results.

The present examples and embodiments are to be considered as illustrative and not restrictive, and the invention is not to be limited to the details given herein, but may be modified within the scope and equivalence of the appended claims.

### Reference numerals

- 1: drug delivery device
- 2: body
- 3: piston rod
- 4A: predetermined area of weakness
- 4B: plastically deformed area
- 5: cartridge
- 6: bung
- 7: cartridge holder
- 8: bearing
- 10: housing
- 11: distal end of the body
- 11A: distal end of the housing
- 11P: distal face of the piston rod
- 12: proximal end of the body and the housing
- 12B: proximal face of the bung
- 14: inductively heatable element
- 15: opening
- 16: axis between the proximal end of the piston rod and thedistal face of the piston rod
- 18: dosing element

## Claims

1. A method for assembling a drug delivery device comprising a body (2) retaining a piston rod (3) and a cartridge (5) retaining a bung (6) and holding a drug, said body (2) having a distal end (11) and a proximal end (12), **characterized in** the following steps:
A) Providing a component defining the distance between the proximal face (12B) of the bung (6) and the proximal end of the piston rod (3),
preferably a plastically deformable piston rod (3), wherein at least a part of said plastically deformable piston rod being manufactured from a plastically deformable material,
B) Engaging the cartridge (5) and the distance defining component,
C) Arranging the cartridge (5) in its most distal position with respect to the distance defining component and determining a distance between the proximal face (12B) of the bung (6) and the proximal end of the distance defining component,
D) Calculating the gap between the proximal face of the bung (6) and the distal end of the piston rod (3) from the distance determined in step C),
E) Adjusting the length of the piston rod (3),
preferably by applying a force on the plastically deformable piston rod (3), at least a part of the piston rod (3) being in a state that allows plastic deformation, so that the piston rod (3) is plastically deformed and an element of the piston rod (3) abuts the bung (6), and by applying conditions in order to bring the plastically deformed piston rod (3) into an altered state wherein no further plastic deformation takes place, wherein the conditions involve altering the physical state of matter and/or the chemical state of matter of at least part of the material of the piston rod,
F) Securing the cartridge (5) to the body (2).

2. The method according to the preceding claim, wherein the applied force results from a movement of the plastically deformable piston rod (3) in distal direction, or a movement of a component being fixed to the plastically deformable piston rod (3) in distal direction, or a movement of the cartridge (5) in proximal direction, or a movement of a component being fixed to the cartridge (5) in proximal direction.

3. The method according to any of the preceding claims, wherein the piston rod (3) is plastically deformed during attachment of the cartridge (5) to the body (2).

4. The method according to any of the preceding claims, wherein prior to applying a force to the plastically deformable piston rod the material of at least part of the piston rod is brought into a plastically deformable state of matter by heating.

5. The method according to any of the preceding claims, wherein the plastically deformable material of at least part of the plastically deformable piston rod comprises a thermoplastic polymer or consists of a thermoplastic polymer.

6. The method according to any of the preceding claims, wherein the altered state of matter of the plastically deformable piston rod is obtained by cooling or by effecting a chemical reaction of the plastically deformable material.

7. The method according to any of the three preceding claims, wherein the material of at least part of the plastically deformable piston rod comprises an inductively heatable compound and wherein the material of at least part of the piston rod is inductively heated prior applying a force to the piston rod to bring it into a plastically deformable state.

8. The method according to any of the preceding claims, wherein the plastically deformable piston rod comprises a first element comprising the plastically deformable material and a second element at least a part of which is manufactured from an inductively heatable material, wherein the first element abuts the second element, and wherein prior to applying a force to the plastically deformable piston rod the plastically deformable material is inductively heated.

9. The method according to claim 1, wherein the distance defining component is a cartridge holder (7).

10. A piston rod (3) contained in an assembly for a drug delivery device having a distal end and a proximal end which are spaced apart in the direction of an axis, said piston rod (3) comprising a predetermined area of weakness (4A), which is plastically deformable upon application of a force in the direction of the axis with respect to the piston rod (3) and comprises inductively heatable material.

11. An assembly for a drug delivery device having a distal end and a proximal end, comprising
- a body (2) retaining a piston rod (3),
- a cartridge (5) retaining a bung (6),
- wherein the cartridge (5) is secured to the body (2)
- and wherein the piston rod (3) abuts the proximal face (12B) of the bung (6), **characterized in that** the piston rod (3) comprises a plastically deformed area (4B), at least a part of said plastically deformed piston rod (3) being obtained from a plastically deformable material, and **in that** the plastically deformed area (4B) is obtained by a plastic deformation of a predetermined area of weakness (4A) of the piston rod (3) upon application of a force in the direction of the axis with respect to the piston rod (3).

12. The assembly according the preceding claim obtained by the method of any of the claims 1 to 9.

13. A drug delivery device comprising the assembly according to any of the two preceding claims.

## Patentansprüche

1. Verfahren zur Montage einer Medikamenten-Verabreichungsvorrichtung, umfassend ein Gehäuse (2), in dem eine Kolbenstange (3) gehalten wird, und eine Kartusche (5), in der ein Pfropfen (6) gehalten wird und die ein Arzneimittel enthält, wobei das Gehäuse (2) ein distales Ende (11) und ein proximales Ende (12) aufweist, **gekennzeichnet durch** die folgenden Schritte:
A) Bereitstellen einer Komponente, die den Abstand zwischen der proximalen Stirnfläche (12B) des Pfropfens (6) und dem proximalen Ende der Kolbenstange (3) definiert,
vorzugsweise eine plastisch verformbare Kolbenstange (3), wobei wenigstens ein Teil der plastisch verformbaren Kolbenstange aus einem plastisch verformbaren Material gefertigt ist,
B) in Eingriff bringen der Kartusche (5) und der einen Abstand definierenden Komponente,
C) Anordnen der Kartusche (5) in ihrer distalsten Stellung bezüglich der einen Abstand definierenden Komponente und Bestimmen eines Abstands zwischen der proximalen Stirnfläche (12B) des Pfropfens (6) und dem proximalen Ende der einen Abstand definierenden Komponente,
D) Berechnen der Lücke zwischen der proximalen Stirnfläche des Pfropfens (6) und dem distalen Ende der Kolbenstange (3) aus dem in Schritt C) bestimmten Abstand,
E) Anpassen der Länge der Kolbenstange (3),
vorzugsweise durch Aufbringen einer Kraft auf die plastisch verformbare Kolbenstange (3), wobei sich wenigstens ein Teil der Kolbenstange (3) in einem Zustand befindet, der eine plastische Verformung gestattet, so dass die Kolbenstange (3) plastisch verformt wird und ein Element der Kolbenstange (3) an den Pfropfen (6) anstößt, und durch Anlegen von Bedingungen, um die plastisch verformte Kolbenstange (3) in eine veränderten Zustand zu bringen, in dem keine weitere plastische Verformung stattfindet, wobei die Bedingungen die Veränderung des physikalischen Aggregatzustands und/oder des chemischen Aggregatzustands wenigstens eines Teils des Materials der Kolbenstange beinhalten,
F) Befestigen der Kartusche (5) an dem Gehäuse (2).

2. Verfahren nach dem vorhergehenden Anspruch, wobei die aufgebrachte Kraft aus einer Bewegung der plastisch verformbaren Kolbenstange (3) in die distale Richtung, oder einer Bewegung einer Komponente, die an der plastisch verformbaren Kolbenstange (3) befestigt ist, in die distale Richtung, oder einer Bewegung der Kartusche (5) in die proximale Richtung, oder einer Bewegung einer Komponente, die an der Kartusche (5) befestigt ist, in die proximale Richtung resultiert.

3. Verfahren nach einem der vorhergehenden Ansprüche, wobei die Kolbenstange (3) während der Befestigung der Kartusche (5) an dem Gehäuse (2) plastisch verformt wird.

4. Verfahren nach einem der vorhergehenden Ansprüche, wobei vor dem Aufbringen einer Kraft auf die plastisch verformbare Kolbenstange das Material wenigstens eines Teils der Kolbenstange durch Erwärmen in einen plastisch verformbaren Aggregatzustand gebracht wird.

5. Verfahren nach einem der vorhergehenden Ansprüche, wobei das plastisch verformbare Material wenigstens eines Teils der plastisch verformbaren Kolbenstange ein thermoplastisches Polymer umfasst oder aus einem thermoplastischen Polymer besteht.

6. Verfahren nach einem der vorhergehenden Ansprüche, wobei der geänderte Aggregatzustand der plastisch verformbaren Kolbenstange durch Kühlen oder durch Herbeiführen einer chemischen Reaktion des plastisch verformbaren Materials erhalten wird.

7. Verfahren nach einem der drei vorhergehenden Ansprüche, wobei das Material wenigstens eines Teils der plastisch verformbaren Kolbenstange eine induktiv erwärmbare Verbindung umfasst und wobei das Material wenigstens eines Teils der Kolbenstange induktiv erwärmt wird, bevor eine Kraft auf die Kolbenstange aufgebracht wird, um sie in einen plastisch verformbaren Zustand zu bringen.

8. Verfahren nach einem der vorhergehenden Ansprüche, wobei die plastisch verformbare Kolbenstange ein erstes Element, welches das plastisch verformbare Material umfasst, und ein zweites Element umfasst, von dem wenigstens ein Teil aus einem induktiv erwärmbaren Material gefertigt ist, wobei das erste Element an das zweite Element anstößt, und wobei vor dem Aufbringen einer Kraft auf die plastisch verformbare Kolbenstange das plastisch verformbare Material induktiv erwärmt wird.

9. Verfahren nach Anspruch 1, wobei die einen Abstand definierende Komponente ein Kartuschenhalter (7) ist.

10. Kolbenstange (3), die in einer Anordnung für eine Medikamenten-Verabreichungsvorrichtung enthalten ist, mit einem distalen Ende und einem proximalen Ende, die in Richtung einer Achse voneinander beabstandet sind, wobei die Kolbenstange (3) einen vorbestimmten Schwächungsbereich (4A) umfasst, der bei Aufbringen einer Kraft in Richtung der Achse bezüglich der Kolbenstange (3) plastisch verformbar ist und induktiv erwärmbares Material umfasst.

11. Anordnung für eine Medikamenten-Verabreichungsvorrichtung mit einem distalen Ende und einem proximalen Ende, umfassend:
- ein Gehäuse (2), in dem eine Kolbenstange (3) gehalten wird,
- eine Kartusche (5), in der ein Pfropfen (6) gehalten wird,
- wobei die Kartusche (5) an dem Gehäuse (2) befestigt ist
- und wobei die Kolbenstange (3) an der proximalen Stirnfläche (12B) des Pfropfens (6) anstößt, **dadurch gekennzeichnet, dass** die Kolbenstange (3) einen plastisch verformten Bereich (4B) umfasst, wobei wenigstens ein Teil der plastisch verformten Kolbenstange (3) aus einem plastisch verformbaren Material erhalten wird, und dass der plastisch verformte Bereich (4B) durch plastische Verformung eines vorbestimmten Schwächebereichs (4A) der Kolbenstange (3) durch Anwendung einer Kraft in Richtung der Achse in Bezug auf die Kolbenstange (3) erhalten wird.

12. Anordnung nach einem der vorhergehenden Ansprüche, erhalten durch das Verfahren nach einem der Ansprüche 1 bis 9.

13. Medikamenten-Verabreichungsvorrichtung, umfassend die Anordnung nach einem der zwei vorhergehenden Ansprüche.

## Revendications

1. Procédé d'assemblage d'un dispositif d'administration de médicaments comprenant un corps (2) maintenant une tige (3) de piston et une cartouche (5) maintenant un bouchon (6) et contenant un médicament, ledit corps (2) ayant une extrémité distale (11) et une extrémité proximale (12), **caractérisé par** les étapes consistant à :
A) fournir un composant définissant la distance entre la face proximale (12B) du bouchon (6) et l'extrémité proximale de la tige (3) de piston, de préférence une tige (3) de piston déformable plastiquement, au moins une partie de ladite tige de piston déformable plastiquement étant fabriquée à partir d'une matière plastiquement déformable,
B) mettre en prise la cartouche (5) et le composant définissant la distance,
C) disposer la cartouche (5) dans sa position la plus distale par rapport au composant définissant la distance et déterminer la entre la face proximale (12B) du bouchon (6) et l'extrémité proximale du composant définissant la distance,
D) calculer l'intervalle entre la face proximale du bouchon (6) et l'extrémité distale de la tige (3) de piston à partir de la distance déterminée à l'étape C),
E) régler la longueur de la tige (3) de piston, de préférence en appliquant une force sur la tige (3) de piston déformable plastiquement, au moins une partie de la tige (3) de piston se trouvant dans un état qui permet une déformation plastique, de sorte que la tige (3) de piston est déformée plastiquement et un élément de la tige (3) de piston bute contre le bouchon (6), et en appliquant des conditions propres à amener la tige (3) de piston déformée plastiquement dans un état modifié dans lequel aucune autre déformation plastique n'a lieu, dans lequel les conditions impliquent de modifier l'état physique de matière et/ou l'état chimique de matière d'au moins une partie du matériau de la tige de piston,
F) bien fixer la cartouche (5) au corps (2).

2. Procédé selon la revendication précédente, dans lequel la force appliquée résulte d'un mouvement dans la direction distale de la tige (3) de piston déformable plastiquement, ou d'un mouvement dans la direction distale d'un composant fixé à la tige (3) de piston déformable plastiquement, ou d'un mouvement dans la direction proximale de la cartouche (5), ou d'un mouvement dans la direction proximale d'un composant fixé à la cartouche (5).

3. Procédé selon l'une quelconque des revendications précédentes, dans lequel la tige (3) de piston est déformée plastiquement pendant la fixation de la cartouche (5) au corps (2).

4. Procédé selon l'une quelconque des revendications précédentes, dans lequel, avant d'appliquer une force à la tige de piston déformable plastiquement, on amène le matériau d'au moins une partie de la tige de piston dans un état de matière déformable plastiquement en le chauffant.

5. Procédé selon l'une quelconque des revendications précédentes, dans lequel le matériau déformable plastiquement d'au moins une partie de la tige de piston déformable plastiquement comprend un polymère thermoplastique ou est constitué d'un polymère thermoplastique.

6. Procédé selon l'une quelconque des revendications précédentes, dans lequel on obtient la modification de l'état de matière de la tige de piston déformable plastiquement en la refroidissant ou en effectuant une réaction chimique sur le matériau déformable plastiquement.

7. Procédé selon l'une quelconque des trois revendications précédentes, dans lequel le matériau d'au moins une partie de la tige de piston déformable plastiquement comprend un composé pouvant être chauffé par induction et dans lequel le matériau d'au moins une partie de la tige de piston est chauffé par induction avant l'application d'une force à la tige de piston pour l'amener dans un état déformable plastiquement.

8. Procédé selon l'une quelconque des revendications précédentes, dans lequel la tige de piston déformable plastiquement comprend un premier élément comprenant le matériau déformable plastiquement et un second élément dont au moins une partie est fabriquée à partir d'un matériau pouvant être chauffé par induction, dans lequel le premier élément est en appui sur le second élément, et dans lequel avant d'appliquer une force à la tige de piston déformable plastiquement on chauffe par induction le matériau déformable plastiquement.

9. Procédé selon la revendication 1, dans lequel le composant définissant la distance est un support (7) de cartouche.

10. Tige (3) de piston incluse dans un ensemble pour un dispositif d'administration de médicaments ayant une extrémité distale et une extrémité proximale qui sont espacées dans la direction d'un axe, ladite tige (3) de piston comprenant une zone prédéterminée de faiblesse (4A), qui est déformable plastiquement quand on applique une force dans la direction de l'axe par rapport à la tige (3) de piston et comprend un matériau chauffable par induction.

11. Ensemble pour un dispositif d'administration de médicaments ayant une extrémité distale et une extrémité proximale, comprenant :
- un corps (2) maintenant une tige (3) de piston,
- une cartouche (5) maintenant un bouchon (6),
- dans lequel la cartouche (5) est solidement fixée au corps (2)
- et dans lequel la tige (3) de piston bute contre la face proximale (12B) du bouchon (6),
**caractérisé en ce que** la tige (3) de piston comprend une zone (4B) déformée plastiquement, au moins une partie de ladite tige (3) de piston déformée plastiquement étant obtenue à partir d'un matériau déformable plastiquement, et **en ce que** la zone (4B) déformée plastiquement est obtenue par une déformation plastique d'une zone prédéterminée de faiblesse (4A) de la tige (3) de piston sur application d'une force dans la direction de l'axe par rapport à la tige (3) du piston.

12. Ensemble selon la revendication précédente, obtenu par le procédé selon l'une quelconque des revendications 1 à 9.

13. Dispositif d'administration de médicaments comprenant l'ensemble selon l'une quelconque des deux revendications précédentes.
